# EUROPEAN PATENT APPLICATION

(11) **EP 1 741 463 A1**
(43) Date of publication of application: **10.01.2007**
(21) Application number: 05388057.1
(22) Date of filing: 05.07.2005
(51) Int. Cl.: A61M 25/00, A61L 29/04

(54) **A guiding and an embolization catheter**

(71) Applicant: Millimed A/S, 4000 Roskilde (DK)
(72) Inventor: Jensen, Christian, 2860 Soborg (DK); Schoedt, Mikkel Tonder, 4000 Roskilde (DK); Glejbol, Kristian, 2600 Glostrup (DK); Petersen, Steen Guldager, 2610 Rodovre (DK)
(74) Representative: Inspicos A/S

(57) **Abstract**

A guiding catheter defines a passageway and comprises a flexible inner tube extending the entire length of the catheter, and an outer tube which defines a flexible distal portion and a rigid proximal portion having a length of at least 40 cm. The catheter is suitable for employment in order to e.g. block blood supply to regions of tumors and for the treatment of aneurysms.

## Description

### Technical field

The present invention relates to a guiding catheter and an embolization catheter suitable for e.g. employment in order to block blood supply to regions of tumors or for the treatment of aneurysms. In particular the invention relates to a so-called "micro catheter".

### Background of the invention

It is known to provide an artificial embolism to e.g. control an internal bleeding, relieve pressure in a vessel wall at an aneurysm or prevent blood supply to tumors. For example, an occlusive wire coil and delivery system for positioning the coil may be applied. Alternatively, the artificial embolism may be provided by an inflatable balloon or by injection of a coagulative substance. Such embolization devices or coagulative substances may be guided to a treatment site within the human body, e.g. a cerebral area, by means of a catheter, through which the embolization device in question is maneuvered or conveyed to the treatment site. Typically, the catheter is entered into the vascular system of the patient at the groin, thus requiring a length of the catheter of 80 - 150 cm.

It is generally desired that medical devices for insertion into the vascular system of a living being meet certain physical requirements. For example, the medical devices must be able to conform to an often tortuous passage to the treatment site while being sufficiently rigid to enable secure insertion. Furthermore, the surface of such medical devices should be hydrophilic and have a low surface friction in order to facilitate introduction. Medical devices which are intended to release pharmaceutical active substances once inserted into the vascular system of a living being may be covered or coated with appropriate pharmaceutical compounds.

In the prior art, various medical devices, including balloons, stents and catheters, as well as methods for their manufacture have been proposed. US patent No. 6,030,371 discloses a method for non-extrusion manufacturing of catheters that can be used to produce catheters having a simple or complex configuration. A polymer material in a particulate preform is applied in a layer over an outer surface of a core member. By applying the layer in a particulate preform, a composition of the polymer material can be varied continuously as it is being applied to provide a variable hardness over the length of the catheter. A fibrous reinforcement can be used having a constant or variable pitch, and a constant or variable number of fibers and fiber types may be employed.

In order to improve desirable manipulation of the catheter, guiding catheters can have several composite features such as a softer, more malleable tip (located approximately in the distal eight to ten centimeter range) to enhance its utility and movement with the remaining portion of the guiding catheter being harder to provide the necessary torsional rigidity and structural integrity. This is generally achieved by using a material that is of higher durometer in the guiding catheter body than the distal tip section.

### Summary of the invention

It is an object of embodiments of the present invention to provide a guiding catheter and an embolization catheter with improved structural characteristics in order to enhance its utility and movement within the vascular system of a living being.

In a first aspect, the invention provides a guiding catheter defining a passageway and comprising a flexible inner tube extending the entire length of the catheter, and an outer tube defining a flexible distal portion, and a rigid proximal portion having a length of at least 40 cm.

It is desirable to have a catheter defining a passageway, the catheter being sufficiently flexible so as to allow the distal end thereof to be guided in various body passages. On the other hand, when the catheter is rotated in a passage it must possess torsional rigidity so as to enhance the structural integrity of the proximal portion of the catheter in order to facilitate the rotation.

The flexible inner tube may be made of e.g. polytetrafluorethylene (PTFE), such as Teflon^{®}, or fluorinated ethylene-propylene (FEP). The outer tube may be made from e.g. stainless steel. The rigid portion may be a rigid tube made from e.g. stainless steel and the flexible portion may be a braided tube or it may be formed as a coil, the flexible portion being made of e.g. a stainless steel thread or a platinum thread. The outer tube may be made in one piece, the rigid portion being a stainless steel tube eventually comprising a helical groove which may be deeper and/or denser at the flexible portion in order to ensure flexibility of this portion. If the flexible portion comprises a braided tube, the flexible distal portion may be braided with a pitch that varies over the length of the distal portion, so that the pitch is larger near a proximal end of the distal portion than near a distal end thereof, i.e. the angel of the threads relative to the axis of the catheter being variable relative to the length of the catheter, the distal portion of the flexible portion having a steeper braiding than the proximal portion thereof, whereby the flexibility of the flexible part may be higher at the distal portion of the flexible portion than of the proximal portion thereof.

As an alternative, the flexible portion of the outer tube may be formed as a coil. If being formed as a coil the pitch thereof may also be variable ensuring a variable flexibility over the length of the flexible portion, the distal portion thereof being more flexible than the proximal portion. A braided flexible portion of the outer tube may result in a catheter which is easier to rotate within the vascular system than if the flexible portion is formed as a coil, since the braided form is more stabile to rotational movements of the catheter. On the contrary, a flexible portion in the form of a coil may result in a catheter which is more resistant to kink, since a braided portion more often may fold transversely to the length of the catheter than may be the fact for a coil formed flexible portion.

The rigid portion may alternatively and/or additionally be made from polymer fibers being spun around the flexible inner tube. Rigidity may be achieved by applying the tube to vacuum subsequently to the spinning process. The spun fibers are thereby applied to a shrinking process which may result in the requested rigidity.

The outer tube may be covered with a coating of nanofibers, which coating may be applied using an electrospinning process and/or a hybrid electrospinning process. The latter can be seen as an electrospinning process configured to apply a stream of gas as a supplement to the electrical field applied in a conventional electrospinning process. Using these processes, the electrical field, eventually supplemented with a stream of gas conveys the dissolved polymer constituting the coating toward the medical device to be coated. Alternatively and/or additionally, the coating may be applied using a dipping, soaking and/or spraying process in order to apply the nanofibers to the medical device.

US patent No. 4,323,525 discloses a process for electrostatically spinning a fiber forming material. The process of electrostatic spinning involves the introduction of a liquid into an electric field whereby the liquid is caused to produce fibers. The spun fiber is collected on a removable sheath on a rotating mandrel. The sheath is electroconductive. The tubular spun fiber product is separated from the sheath.

Electrospinning comprises a process of formation of fibers (nanofibers) from a fluid exploiting the interactions between surface tension and the electrostatic forces exerted in the fluid surface by an applied electrostatic field. When an electrostatic field is applied to e.g. a droplet of conducting fluid, the fluid surface will experience forces both due to the surface tension as well as due to the applied electrostatic field. At comparably low electrostatic fields the surface tension of the fluid will dominate and the droplet will essentially remain spherical. When the electrostatic field is increased the droplet will become increasingly deformed by electrostatic forces until the surface of the droplet becomes unstable and a tiny stream of liquid is ejected from the surface. The material in this stream eventually solidifies, thus forming a fiber.

It should be understood that the term electrospinning comprises a process wherein particles emerge from a source kept at a certain, preferably constant, electric potential. By applying an electrode kept at another certain, preferably constant, electrical potential, an electrical field exists. The particles will under influence of this electrical field be directed toward the medical device.

The art of producing nanofibers has developed considerably in recent years. US patent No. 6,382,526 discloses a process and apparatus for the production of nanofibers, which process and apparatus are useful in the method according to the present invention, and US patent No. 6,520,425 discloses a nozzle for forming nanofibers. It should be understood that processes and apparatuses of the aforementioned US patents may be applicable in the method according to the present invention, but that the scope of protection is not restricted to those processes and apparatuses.

The nanofibers may be prepared from a polymer, i.e. various polymer-based materials and composite matrixes thereof, including polymer solutions and polymer melts. Applicable polymers are, e.g. polyamides including nylon, polyurethanes, fluoropolymers, polyolefins, polyimides, polyimines, (meth)acrylic polymers, and polyesters, as well as suitable copolymers.

In using an electrospinning process for the preparation of the nanofibers, the solvent for the polymer may be any solvent capable of dissolving the polymer and providing a conducting fluid capable of being electrospun. Typical solvents include a solvent selected from N,N-Dimethyl formamide (DMF), tetrahydrofuran (THF), methylene chloride, dioxane, methanol, ethanol, higher alcohols, chloroform, water and mixtures hereof. The conducting fluid may optionally comprise a pharmaceutically active substance, i.e. a material releasing therapeutic agents when subjected to bodily fluids.

A coating covering the outer tube may comprise a first portion, which is hydrophilic, and a second portion, which is loaded with a pharmaceutically active substance. A first portion being hydrophilic may result in an outer surface having a low friction thereby facilitating introduction of the catheter in the vascular system. For example, a first portion being hydrophilic may be made of hydrophilic polymer, e.g. polyacrylic acids and copolymers, polyethylene oxides, polyN-vinyl lactams such as polyvinyl pyrrolidone, etc.

In other embodiments of the invention, a low surface friction may be achieved by applying a hygroscopic material as a fiber forming material in the electrospinning process. Accordingly, once introduced in the vascular system, the hygroscopic electrospun material absorbs body fluid, resulting in a hydrophilic low-friction surface. A hygroscopic surface may for example be achieved with a polyurethane or a polyacrylic acid material.

Furthermore, a barrier layer may be provided as a coating on the outer surface layer in order to ensure that contact between the outer surface layer and blood is delayed until the catheter is in place. The barrier layer may be formed of a biodegradable polymer which dissolves or disintegrates.

Many pharmaceutically active substances may be chosen for loading the second portion, among these human growth factors or analogies to these, antiseptic agents, agents suppressing inflammatory tissue response, heparin or chemotherapeutical agents. These pharmaceutically active substances among others, or mixtures hereof, may be dissolved in the spinning solution, thus giving the second portion of the coated surface drug releasing properties. Basically two types of pharmaceutically active substances exist. In one case the pharmaceutically active substance in itself dissolves into monomers or dimmers that can diffuse to the surrounding tissue. An alternative approach is to include materials that degenerate thus releasing pharmaceutically active substances. One example of such a material is nitric oxide modified linear poly(etihylenimine) diazeniumdiolate as described in US patent No. 6,737,447. Upon subjection to neutral or acidic water this material will release nitric oxide.

Various nitric oxide donor components, pharmaceutical compositions containing such nitric oxide donor components and polymeric compositions capable of releasing nitric oxide have also been proposed in the prior art. For example, US patent No. 6,737,447 B1 discloses a medical device comprising at least one nanofiber of a linear poly(etihylenimine) diazeniumdiolate forming a coating layer on the device. US 6,737,447 mentions the possibility of depositing the polymer by an electrospinning process.

The first portion of the coating may be proximal with respect to the second portion in order to ensure that the pharmaceutically active substance loaded in the second portion is delivered to the treatment site, i.e. the outermost point of the passageway defined by the guiding catheter. The first portion may cover the rest of the catheter in order to facilitate introduction thereof. Alternatively, the first portion may cover the outer tube fully, since the second portion may be both hydrophilic and be loaded with pharmaceutically active substances.

The pharmaceutically active substance may be provided in the form of biodegradable beadings distributed between the nanofibers, the beadings being capable of releasing the pharmaceutically active substance and, in the case of biodegradable beadings, to degrade following release. Such beadings, which are described in more detail in International patent application No. PCT/DK2004/000560 which is hereby incorporated by reference in its entirety, may penetrate into the tissue at the treatment site and release the pharmaceutically active substance there. Alternatively, they may be of a size which is so small that they may be transported away from the treatment site, e.g. with the flow of blood.

The pharmaceutically active substance may comprise nitric oxide. Various nitric oxide (NO) donor compounds and polymeric compositions capable of releasing nitric oxide have been proposed in the prior art, e.g. US 5,691,423, US 5,962,520, US 5,958,427, US 6,147,068, and US 6,737,447 B1, all of which are incorporated herein by reference.

In embodiments of the present invention, the nanofibers are made from polymers which have nitric oxide donors (e.g. a diazeniumdiolate moiety) covalently bound thereto. Polyimines represent a diverse group of polymer which may have diazeniumdiolate moieties covalently bound thereto. Polyimines include poly(alkylenimines) such as poly(ethylenimines). For example, the polymer may be a linear poly(ethylenimine) diazeniumdiolate (NONO-PEI) as disclosed in US 6,737,447 which is hereby incorporated by reference. The loading of the nitric oxide donor onto the linear poly(ethylenimine) (PEI) can be varied so that 5-80%, e.g. 10-50%, such as 33%, of the amine groups of the PEI carry a diazeniumdiolate moiety. Depending on the applied conditions, the linear NONO-PEI can liberate various fractions of the total amount of releasable nitric oxide.

Polyamines with diazeniumdiolate moieties (in particular poly(ethylenimine) diazeniumdiolate) may advantageously be used as a polymer for the coating with nanofibers because such polymers typically have a suitable hydrophilicity and because the load of diazeniumdiolate moieties (and thereby the load of latent NO molecules) can be varied over a broad range.

Nitric oxide may relax or prevent arterial spasm once the guiding catheter is in place. Nitric oxide may also inhibit the aggregation of platelets and reduces smooth muscle proliferation, which reduces restenosis. When delivered directly to a particular site, it has been shown to prevent or reduce inflammation at the site where medical personnel have introduced foreign objects or devices into the patient.

The outer tube may further comprise an acidic agent. Acidic agents may enhance the release of pharmaceutically active substances. As an example, the rate of nitric oxide (NO) liberation highly depends on the pH of the media. Thus, by addition of various amounts of an acid, the rate of NO liberation can be controlled. E.g. the half-life of NO liberation at pH = 5.0 is approximately 20 minutes whereas at pH = 7.4 the half-life is approximately 10 hours. As examples, Ascorbic Acid and Lactic Acid can be used as acidic agents for enhancing release of pharmaceutically active substances.

The guiding catheter may further define a tip at a distal end thereof, which tip may be of the length of between 2 and 5 mm. The guiding catheter may be used for delivering an embolization agent to the treatment site, the embolization agent being employed in order to e.g. block blood supply to regions of tumors or in the treatment of aneurysms. Since cyanoacrylate based adhesives may be used as embolization agents, the tip may be made from a material which is insoluble in cyanoacrylate adhesives. Appropriate materials for the tip may be e.g. polyethylene or PTFE, such as Teflon^{®}. If the flexible inner tube is made of PTFE, the tip may be made as an integrated part thereof, as the inner tube may be longer that the outer tube and folded over the outer tube, thus defining the tip.

An x-ray opaque sleeve may facilitate the positioning of the catheter at the treatment site, and consequently an x-ray opaque sleeve may be fitted around the outer tube near the tip, and the tip may be fitted around the sleeve and the distal end of the outer tube. Viewing the patient on an x-ray imaging screen while introducing the guiding catheter, the positioning of the tip at the treatment site may be more precise and easier obtained.

In a second aspect, the present invention provides an embolization catheter comprising a main tubular portion defining a passageway, and a tip defining a distal end of the main tubular portion, the tip being made from a material which is insoluble in cyanoacrylate adhesives.

It should be understood that the abovementioned features may also be applicable for the tip of the embolization catheter. The tip may be made of nanofibers, e.g. electrospun nanofiber.

The main tubular portion may comprise a flexible inner tube extending the entire length of the catheter and an outer tube defining a flexible distal portion and a rigid proximal portion having a length of at least 40 cm. The features of the abovementioned guiding catheter may also be applicable for the main tubular portion of the embolization catheter.

In a third aspect, the present invention provides a guiding catheter comprising a main tubular portion defining a passageway and having a differing rigidity along its length. Such different properties may be arrived at by e.g. employing different fiber-forming materials for different sections and/or by changing production parameters. In case of the use of an electrospinning process for the production of the guiding catheter, voltage of the electrodes, distance between high-voltage and low-voltage electrodes, rotational speed of the guiding catheter, electrical field intensity, corona discharge initiation voltage or corona discharge current may be employed.

It should be understood that the abovementioned features may also be applicable for the guiding catheter having a differing rigidity along its length.

### Brief description of the drawings

Embodiments of the invention will now be further described with reference to the drawings, in which:
Fig. 1 is a sectional view of a guiding catheter.
Fig. 2 illustrates a guiding catheter having a differing rigidity along its length.
Fig. 3 is a sectional view of a guiding catheter having a differing rigidity along its length.

### Detailed description of the drawings

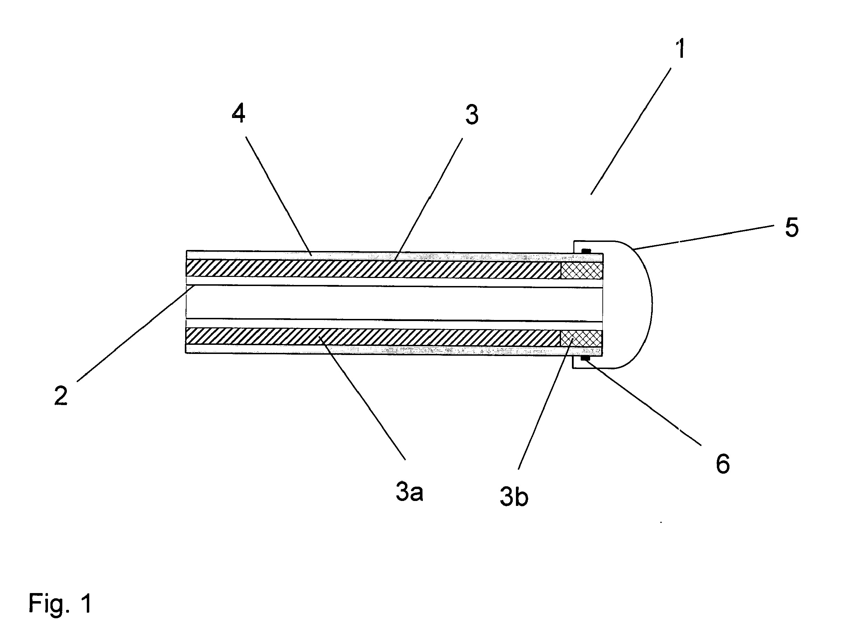

Fig. 1 shows a sectional view of a guiding catheter 1 having a flexible inner tube 2 extending the entire length of the catheter 1 and an outer tube 3 defining a flexible distal portion 3a and a rigid proximal portion 3b. The outer tube 3 is coated with a coating of electrospun nanofibers 4. A tip 5 is provided at the distal end of the guiding catheter 1. Furthermore, an x-ray opaque sleeve 6 is fitted around the outer tube 3.

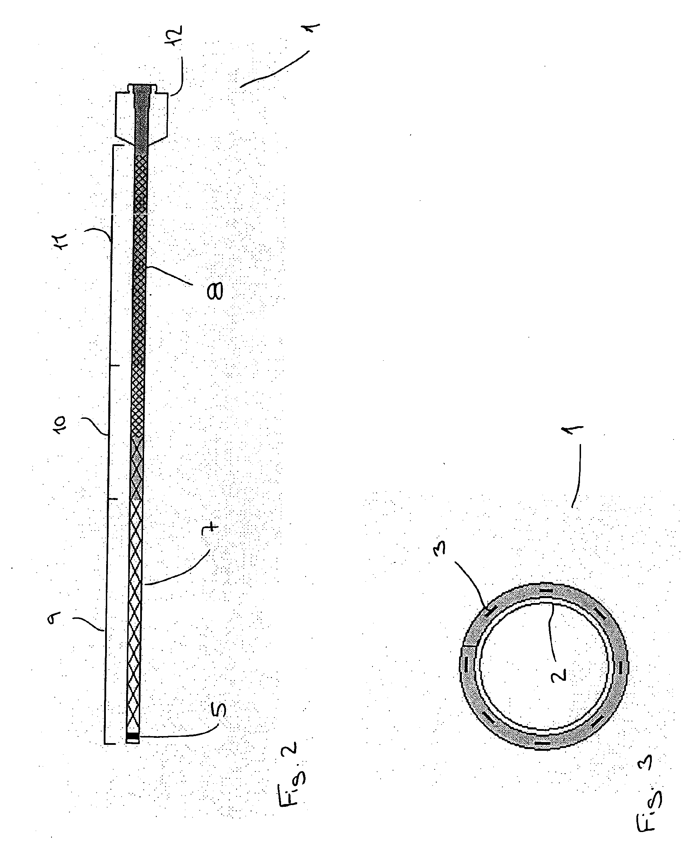

Fig. 2 shows a guiding catheter 1 having a differing rigidity along its length and Fig. 3 is a sectional view thereof. The sectional view in Fig. 3 shows a flexible inner tube 2 having a wall thickness of 0.001" to 0.002" and an inner diameter of 0.016" to 0.028". The outer tube 3 comprising a braided stainless steel wire of 0.003" x 0.0005" (flat wire) is enclosing the inner tube 2. The braiding may vary in density, i.e. a wire may cross another wire from 10 to 300 times per inch when measured along the length of the guiding catheter 1, thereby allowing for a braiding with a pitch that varies over the length thereof. The varying pitch is shown in Fig. 2, where a sparse braiding 7 at the distal portion of the guiding catheter 1 allows for more flexibility than at the proximal portion comprising a more dense braiding 8. Alternatively, the dense braiding 8 may be replaced by a stainless steel tube. The outer layer further comprises a soft polymer e.g. polyurethane or a polyamine, such as PEBAX^{®}. The sections 9, 10 and 11 show a polymer coating having different rigidity along the length of the guiding catheter 1, thereby allowing for a catheter having different flexibility along its length.

A tip 5 provided at the distal end of the guiding catheter 1 is shown at Fig. 2. The tip contains a metal band of Pt Ir alloy in order to make the guiding catheter detectable by x-ray. The hub 12 at the proximal end of the guiding catheter 1 is made of e.g. a methylpentene based polyolefin, such as Polymethylpentene (e.g. TPX^{®}).

## Claims

1. A guiding catheter defining a passageway and comprising:
- a flexible inner tube extending the entire length of the catheter; and
- an outer tube defining:
- a flexible distal portion; and
- a rigid proximal portion having a length of at least 40 cm.

2. The guiding catheter of claim 1, wherein the outer tube is covered by a coating of nanofibers.

3. The guiding catheter of claim 2, wherein said coating comprises a first portion, which is hydrophilic, and a second portion, which is loaded with a pharmaceutically active substance.

4. The guiding catheter of claim 3, wherein said first portion is proximal with respect to said second portion.

5. The guiding catheter according of claim 3, wherein said pharmaceutically active substance comprises nitric oxide.

6. The guiding catheter of claim 1, wherein the flexible distal portion is braided with a pitch that varies over the length of the distal portion, so that the pitch is larger near a proximal end of the distal portion than near a distal end thereof.

7. The guiding catheter of claim 1, further defining a tip at a distal end thereof, the tip being made from a material which is insoluble in cyanoacrylate adhesives.

8. The guiding catheter of claim 7, wherein the length of the tip is between 2 and 5 mm.

9. The guiding catheter of claim 7, wherein the tip is made of polyethylene.

10. The guiding catheter of claim 7, wherein an x-ray opaque sleeve is fitted around the outer tube near the tip, and wherein the tip is fitted around said sleeve and the distal end of the outer tube.

11. An embolization catheter comprising:
- a main tubular portion defining a passageway; and
- a tip defining a distal end of the main tubular portion, the tip being made from a material which is insoluble in cyanoacrylate adhesives.

12. The embolization catheter of claim 11, wherein the length of the tip is between 2 and 5 mm.

13. The embolization catheter of claim 11, wherein the tip is made of polyethylene.

14. The embolization catheter of claim 11, wherein an x-ray opaque sleeve is fitted around the main tubular portion near the tip thereof, and wherein the tip is fitted around said sleeve and the distal end of the main tubular portion.

15. The embolization catheter of claim 11, wherein the tip is made of nanofibers.

16. The embolization catheter of claim 11, wherein the main tubular portion comprises a flexible inner tube extending the entire length of the catheter and an outer tube defining:
- a flexible distal portion; and
- a rigid proximal portion having a length of at least 40 cm.

17. The embolization catheter of claim 11, wherein the main tubular portion is covered by a coating of nanofibers.

18. The embolization catheter of claim 17, wherein said coating comprises a first portion, which is hydrophilic, and a second portion, which is loaded with a pharmaceutically active substance.

19. The embolization catheter of claim 18, wherein said first portion is proximal with respect to said second portion.

20. The embolization catheter of claim 18, wherein said pharmaceutically active substance comprises nitric oxide.

21. The embolization catheter of claim 16, wherein the flexible distal portion is braided with a pitch that varies over the length of the distal portion, so that the pitch is larger near a proximal end of the distal portion than near a distal end thereof.

22. A guiding catheter comprising a main tubular portion defining a passageway and having a differing rigidity along its length.
